# EUROPEAN PATENT APPLICATION

(11) **EP 4 772 606 A1**
(43) Date of publication of application: **08.07.2026**
(21) Application number: 24858434.4
(22) Date of filing: 22.08.2024
(51) Int. Cl.: C12M 1/38, C12M 1/34, C12M 1/00, C12Q 1/686

(54) **LASER-INDUCED GRAPHENE AND USE THEREOF IN PCR DETECTION**

(30) Priority: 30.08.2023 CN 202311102706; 01.09.2023 CN 202311118666
(71) Applicant: Beijing Xinchang Technology Ltd, Beijing 102402 (CN)
(72) Inventor: WANG, Yang, Beijing 102402 (CN)
(74) Representative: Gille Hrabal Partnerschaftsgesellschaft mbB Patentanwälte
(86) International application number: PCT/CN2024/113961
(87) International publication number: WO 2025/044892

(57) **Abstract**

The present invention relates to a laser-induced graphene and its application in PCR detection, as well as a disc-type nucleic acid detection microfluidic chip. The laser-induced graphene of the present invention possesses efficient electrothermal conversion capability, rapid and precise temperature response under applied voltage, fast temperature change capability, and efficient heat transfer properties, enabling precise regulation of liquid temperature to meet the temperature accuracy requirements of the PCR process. The present invention also relates to a disc-type nucleic acid detection microfluidic chip, which has the following advantages: 1. High level of integration, with low reagent and sample consumption, requiring only a drop of blood or other body fluid from the test subject to achieve sample input and result output; 2. Compact size, easy to carry, requiring no instruments or equipment, and only needing a finger to turn the handle, making it easy to operate with low technical requirements for laboratory personnel; 3. With the aid of immunochromatographic test strips, detection results can be read manually within minutes, providing intuitive result interpretation without the need for any instruments; 4. Low cost, easy to process, and suitable for mass production.

## Description

### Technical Field

The present invention belongs to the technical field of detection or analysis of nucleic acids, pathogens, microorganisms, etc., and specifically relates to a type of laser-induced graphene (LIG) and its application in PCR detection, as well as a disc-type microfluidic chip for nucleic acid detection.

### Background Technology

Polymerase Chain Reaction (PCR) is a molecular biology technique used for the amplification of specific DNA fragments. Due to its advantages of speed, sensitivity, specificity, and accuracy, it is widely applied in the rapid detection and identification of bacteria, fungi, viruses, and parasites, as well as in the early screening and diagnosis of diseases. PCR utilizes the principle that DNA denatures into single strands at a high temperature of 95°C *in vitro.* At a low temperature (typically around 60°C), primers bind to the single strands according to the principle of complementary base pairing. The temperature is then adjusted to the optimal reaction temperature for DNA polymerase (around 72°C), where the polymerase synthesizes the complementary strand in the 5' to 3' direction. Based on the principle of the PCR reaction, a complete PCR process involves three stages: Denaturation (90-95°C), Annealing (40-60°C), and Extension (70-75°C). These stages are repeated cyclically for 45 times. Therefore, during the PCR process, the heating and cooling rate of the temperature control module determines the speed of the PCR reaction. Increasing the heating and cooling rate of the temperature control module can effectively accelerate the PCR reaction speed.

Traditional PCR instruments require specialized thermal cycling equipment and often use auxiliary cooling methods, such as fans, to provide the temperatures required for the PCR reaction. For example, CN110551622A discloses a rapid PCR reaction chip and a rapid fluorescence quantitative detector, comprising a panel and a bottom film. The bottom surface of the panel is provided with at least one reaction chamber, which does not penetrate the panel. Sample injection holes and vent holes are opened on both sides of the reaction chamber, connecting to the top surface of the panel. The bottom film is attached to the bottom surface of the panel, and the panel is made of a light-transmitting material. When the PCR chip is placed on a temperature control module, the bottom film is in close contact with the module. After sample injection, the PCR reaction solution in the reaction chamber above the bottom film is separated from the temperature control module only by the bottom film layer. The cooling or heating capacity of the temperature control module can quickly pass through the bottom film and be conducted to the PCR reaction solution. CN107603874A discloses a microfluidic PCR detection system, comprising a microfluidic PCR chip, a sample loading device, a thermal cycling device, and a fluorescence collection device. The microfluidic PCR chip includes a base part, a chamber part, a sealing cover, and a gas inlet. The sample loading device is used for adding the sample to be tested at the hydrophilic unit, forming a sealed oil layer above the added sample, and injecting gas into the reaction chamber through the gas inlet. The thermal cycling device is used for heating the base to allow the sample to undergo thermal cycling amplification. The fluorescence collection device is used for collecting fluorescence signals from the sample after thermal cycling amplification and can output the collected signals externally.

Such thermal cycling equipment is expensive, bulky, complex in system, requires a 220V power supply, and has a relatively slow heating and cooling rate (4-5°C/s). Consequently, PCR technology is almost exclusively confined to large, centralized clinical laboratories or testing centers, rendering it ineffective for applications in the field of rapid point-of-care testing.

On the other hand, pathogen detection has broad applications in the diagnosis and management of infections. Particularly, the early diagnosis of certain infectious diseases is crucial for identifying the illness and providing correct treatment. Point-of-care and on-site diagnostic decisions also help prevent the spread of epidemics and pandemics. Nucleic acid testing technology enables diagnosis in the early stages of a disease, with the entire diagnostic process taking only a few hours, while providing molecular information about the pathogen. It features a wide application range, high sensitivity, strong specificity, and rapidity, gradually becoming an alternative technology to traditional culture methods and immunoassays.

CN112126587B discloses a nucleic acid detection chip device, a nucleic acid detection chip, and a preparation method thereof. The method includes preparing a modified chip, a reaction chip, and a valve chip, then sealing the reaction chip and the valve chip together with the modified bottom plate to form the nucleic acid detection chip. CN115651807B discloses a nucleic acid detection chip and a nucleic acid detection method. The chip comprises a flow channel layer, an elastic film layer, and an air channel layer stacked in sequence. The flow channel layer is provided with microfluidic channels, a sample injection port group, and a sample discharge port group. It is also sequentially provided with a virus isolation zone, a nucleic acid lysis zone, a nucleic acid distribution zone, an LAMP amplification zone, and a CRISPR detection zone connected via the microfluidic channels. The elastic film layer is correspondingly provided with a connection inlet group and a connection outlet group. The air channel layer is provided with air channels corresponding respectively to the microfluidic channels, the nucleic acid lysis zone, the nucleic acid distribution zone, the LAMP amplification zone, and the CRISPR detection zone. The air channel layer is also correspondingly provided with an introduction port group and a discharge port group.

However, nucleic acid testing is currently performed almost exclusively in large, centralized clinical laboratories. This limited accessibility stems largely from the complexity and high cost of traditional nucleic acid technologies. Commercialized nucleic acid testing mainly utilizes PCR to amplify and detect specific nucleic acid targets. Such systems require not only bulky, specialized thermal cycling equipment and fluorescence detection instruments but also trained personnel for operation. There is a lack of effective point-of-care testing (POCT) devices suitable for the rapid on-site processing, washing, amplification, and detection of nucleic acids.

Microfluidic chip technology utilizes the structure of microchannels to enable controllable liquid flow, integrating the entire detection process, including sampling, dilution, reagent addition, reaction, separation, and detection, onto a single microplatform. When combined with isothermal nucleic acid amplification techniques, it can achieve an integrated and automated process where "samples in, results out." However, due to the complexity of raw samples such as blood, sputum, urine, and tissue, there is a lack of uniform standards for sample preparation. To overcome this challenge, not only does the design and manufacturing of microfluidic devices need to be improved, but also the underlying chemistry and materials for nucleic acid capture and extraction require corresponding research and development. Additionally, in the design of microfluidic chips, it is often necessary to control the directional and sequential flow of liquids, which necessitates the use of centrifuges or external driving pumps for most microfluidic chips. However, eliminating the need for instruments and simplifying the internal structural design of microfluidic chips is also of great significance in resource-limited environments. Therefore, there is a need to develop low-cost, integrated, easy-to-operate, instrument-free nucleic acid analysis equipment that can be used by minimally trained personnel to achieve widespread application of nucleic acid detection.

### Summary of the Invention

To address the above-mentioned problems, the present invention proposes a laser-induced graphene PCR detection device and method. It employs an ultra-fast heating and cooling chip based on laser-induced graphene material to solve the issues of current PCR thermal cyclers, such as high cost, large size, system complexity, the need for a 220V power supply, and slow heating and cooling rates (4-5°C/s).

Furthermore, to address the above problems, the present invention integrates sample processing functions with nucleic acid amplification and detection functions, and develops a low-cost, integrated disc-type microfluidic chip for nucleic acid detection. The chip imitates the appearance of a rotary dial telephone and uses cellulose filter paper as the nucleic acid adsorption material. Only a drop of blood or other body fluid from the subject is required. After sample injection, cells in the body fluid are lysed autonomously, and nucleic acids are bound thereto. Following simple washing, the nucleic acids react with the nucleic acid amplification reaction solution stored in the reservoir to realize nucleic acid amplification. Finally, the results are observed and read via immunochromatographic test strips, color reaction or fluorescent color reaction. Switching between different functions can be achieved by simple rotation, thereby realizing on-site point-of-care detection of pathogens. The invention has the advantages of high integration, low cost, simple operation and visually readable detection results, and possesses important practical application value as well as social and economic benefits for on-site point-of-care nucleic acid detection.

The complete technical solution of the present invention includes:
A method for preparing laser-induced graphene, comprising: reducing a precursor material layer using laser-induced processing.

Further, the method comprises providing a carbon-containing precursor material layer, which is located on a base material layer, and using a laser to scan the surface of the carbon-containing precursor material layer. The localized heat generated by the laser irradiation causes the surface of the precursor material to carbonize, thereby forming graphene.

Further, the precursor material layer and the base material layer can be an integrated single material or separate, different materials.

Preferably, the precursor material is selected from polyimide, phenolic resin, polyethyleneimine, lignocellulose, or wood.

Preferably, the laser used is selected from a carbon dioxide laser or a femtosecond laser.

Preferably, the laser scanning speed is 0.5-500 mm/s, preferably 20-300 mm/s, and more preferably 50-150 mm/s.

Preferably, the laser fluence for the laser-induced processing is greater than or equal to 5.5 J/cm², preferably greater than 5.5 J/cm², more preferably 5.8-80 J/cm², even more preferably 5.8-60 J/cm², and most preferably 5.8-50 J/cm².

Preferably, the base material is selected from polyimide material, polyimide film, polyimide tape, phenolic resin, polyethyleneimine, lignocellulose, or wood.

A laser-induced graphene obtained by the method according to any one of the above.

Use of the above-mentioned laser-induced graphene in PCR detection.

A laser-induced graphene PCR detection device, comprising a PCR reaction chamber and a graphene heating component.

Further, the graphene heating component comprises a laser-induced graphene layer and a base material layer. Preferably, the laser-induced graphene layer is located on the surface of the base material layer and has a thickness of 1-120 µm, preferably 2-100 µm, 3-80 µm, or 5-60 µm.

Further, the laser-induced graphene layer is prepared by reducing a precursor material layer using laser-induced processing, wherein the precursor material layer is located on the surface of the base material layer. Preferably, the laser fluence at the start of the laser-induced processing is 5.5 J/cm².

Further, the laser used includes, but is not limited to, a carbon dioxide laser or femtosecond laser. The base material layer includes, but is not limited to, polyimide material, polyimide film, polyimide tape, phenolic resin, polyethyleneimine, lignocellulose, or wood.

Further, the detection device also comprises a power source and a power control module. The power control module controls the power source to perform on/off cycling operations on the graphene heating component.

Further, the graphene heating component is in contact with the PCR reaction chamber. When powered on, it heats the PCR reaction chamber; when powered off, it allows the PCR reaction chamber to cool, thereby achieving a heating/cooling cycle for the PCR reaction chamber.

A laser-induced graphene PCR detection device, comprising a PCR reaction chamber, a graphene heating component, a power source, and a power control module. The graphene heating component is connected to the power source, and the power source is controlled by the power control module. The power control module controls the power source to perform on/off cycling operations on the graphene heating component. The graphene heating component is in contact with the PCR reaction chamber. When powered on, it heats the PCR reaction chamber; when powered off, it allows the PCR reaction chamber to cool, thereby achieving a heating/cooling cycle for the PCR reaction chamber.

Further, the power source is a low-voltage DC power source.

Further, the power source includes, but is not limited to, a battery, a rechargeable battery, a portable power bank, a mobile phone, a power supply unit, or a 220V mains supply.

Further, the graphene heating component is in the form of a sheet.

Further, the graphene heating component comprises a laser-induced graphene layer and a base material layer. Preferably, the laser-induced graphene layer is located on the surface of the base material layer and has a thickness of 1-120 µm, preferably 2-100 µm, 3-80 µm, or 5-60 µm.

Further, the laser-induced graphene layer is prepared by reducing a precursor material layer using laser-induced processing, wherein the precursor material layer is located on the surface of the base material layer. The laser fluence at the start of the laser-induced processing is 5.5 J/cm².

Further, the laser used includes, but is not limited to, a carbon dioxide laser or femtosecond laser. The base material layer includes, but is not limited to, polyimide material, polyimide film, polyimide tape, phenolic resin, polyethyleneimine, lignocellulose, or wood.

Further, the PCR detection device is a point-of-care rapid detection device or a high-throughput benchtop detection device.

Further, the PCR reaction chamber is a disc-type PCR detection chip. The disc-type PCR detection chip comprises an upper sample introduction layer and a lower detection layer.

Further, the sample introduction layer is located within a top cover layer, and the detection layer is located within a reservoir layer.

A method for performing the PCR detection using the described detection device, wherein the power control module is used to control the power source to perform on/off cycling on the graphene heating component, subjecting the PCR reaction chamber to 1-120 thermal cycles, preferably 1-80 cycles, more preferably 1-50 cycles, for example, 45 cycles.

A PCR detection chip, comprising a top cover layer located above and a reservoir layer located below. The top cover layer is rotatable relative to the reservoir layer. The top cover layer includes a sample inlet for adding a test sample. An adsorbent material for adsorbing the test sample is provided below the sample inlet.

The reservoir layer at least includes a sample addition well, an amplification reaction well, and a product detection well. During the rotation of the top cover layer, the adsorbent material can sequentially enter the sample addition well, the amplification reaction well, and the product detection well. A test strip insertion port is provided within the product detection well.

Further, the top cover layer and the reservoir layer are fastened together to form an overall sealed space. Both the top cover layer and the reservoir layer are circular.

Further, the top cover layer is provided with a rotating handle that can be toggled to realize its rotation.

Further, the product detection well is sealed with a sealing material. The sealing material can melt at a certain temperature, allowing the liquid in the product detection well to fall. Preferably, the sealing material is paraffin wax, which can melt at 60-90°C.

Further, a test strip groove for inserting a detection test strip is provided below the reservoir layer. One end of the test strip groove has a through hole serving as an observation window.

Further, at least one washing well is also included between the sample addition well and the amplification reaction well.

Further, two washing wells are provided, including a first washing well and a second washing well.

Further, relative rotation between the top cover layer and the reservoir layer is achieved via a circular slide track.

Further, the adsorbent material is cellulose filter paper.

Further, the sealing material is paraffin wax, which can melt at 60-90°C.

Further, the materials of the top cover layer and the reservoir layer include, but are not limited to, polymethyl methacrylate (PMMA), epoxy resin, PVC, or PC. The concave-convex structures of the slide track and reaction wells are fabricated using laser etching technology, or by etching the structures on a substrate followed by reverse molding with polydimethylsiloxane (PDMS).

Further, the first washing well and the second washing well are respectively loaded with washing buffer, the amplification reaction well is loaded with nucleic acid amplification reaction buffer or PCR reaction reagents, and the product detection well is loaded with running buffer.

Further, the surface of the top cover layer is marked accordingly corresponding to the sample addition well, the first washing well, the second washing well, and the amplification reaction well of the reservoir layer.

Further, the top cover layer and the reservoir layer are subjected to surface hydrophobic treatment. Specifically, the top cover layer and the reservoir layer are treated with trichloro (1H,1H,2H,2H-perfluorooctyl) silane reagent under vacuum for three hours, allowing the trichloro (1H,1H,2H,2H-perfluorooctyl) silane to form a self-assembled monolayer on the surfaces of the top cover layer and the reservoir layer.

A disc-type microfluidic chip for nucleic acid detection comprises an upper top cover layer and a lower reservoir layer. The top cover layer and the reservoir layer are fastened together to form a sealed space as a whole. Both the top cover layer and the reservoir layer are circular, and the top cover layer is rotatable relative to the reservoir layer. The top cover layer comprises a sample inlet for adding a detection sample, and an adsorbent for adsorbing the detection sample is arranged below the sample inlet. A rotating handle capable of being toggled to realize rotation is arranged on the top cover layer.

The reservoir layer at least includes a sample addition well, an amplification reaction well, and a product detection well. During the rotation of the top cover layer, the adsorbent material can sequentially enter the sample addition well, the amplification reaction well, and the product detection well. A test strip insertion port is provided within the product detection well and is sealed with a sealing material. The sealing material can melt at a certain temperature, allowing the liquid in the product detection well to fall.

A test strip groove for inserting a detection test strip is provided below the reservoir layer. One end of the test strip groove has a through hole serving as an observation window.

Further, at least one washing well is also included between the sample addition well and the amplification reaction well.

Further, two washing wells are provided, including a first washing well and a second washing well.

Further, relative rotation between the top cover layer and the reservoir layer is achieved via a circular slide track.

Further, the adsorbent material is cellulose filter paper.

Further, the sealing material is paraffin wax, which can melt at 60-90°C.

Further, the materials of the top cover layer and the reservoir layer include, but are not limited to, PMMA, epoxy resin, PVC, or PC. The concave-convex structures of the slide track and reaction wells are fabricated using laser etching technology, or by etching the structures on a substrate followed by reverse molding with PDMS.

Further, the first washing well and the second washing well are respectively loaded with washing buffer, the amplification reaction well is loaded with nucleic acid amplification reaction buffer or PCR reaction reagents, and the product detection well is loaded with running buffer.

Further, the surface of the top cover layer is marked accordingly corresponding to the sample addition well, the first washing well, the second washing well, and the amplification reaction well of the reservoir layer.

Further, the top cover layer and the reservoir layer are subjected to surface hydrophobic treatment. Specifically, the top cover layer and the reservoir layer are treated with trichloro (1H,1H,2H,2H-perfluorooctyl) silane reagent under vacuum for three hours, allowing the trichloro (1H,1H,2H,2H-perfluorooctyl) silane to form a self-assembled monolayer on the surfaces of the top cover layer and the reservoir layer.

Further, the method comprises the following steps:
Step 1: Rotate the sample inlet of the top cover layer above the sample addition chamber of the reservoir layer. Use a pipette to inject a certain volume of body fluid onto the surface of the adsorbent through the sample inlet, and wait for a period of time for the body fluid to dry.
Step 2: Toggle the rotating handle of the top cover layer with a finger to rotate the adsorbent to the first washing chamber to wash away impurities on the surface of the adsorbent. In this step, toggling the rotating handle left and right can accelerate the washing speed.
Step 3: Toggle the rotating handle of the top cover layer again to rotate the adsorbent to the second washing chamber for further washing. In this step, toggling the rotating handle left and right can accelerate the washing speed.
Step 4: Toggle the rotating handle of the top cover layer to rotate the adsorbent to the amplification reaction chamber. Then turn on the heating device to raise the temperature to a certain level, so that the reaction buffer in the amplification reaction chamber and the nucleic acid on the adsorbent undergo an amplification reaction. The amplification reaction includes, but is not limited to, isothermal amplification reaction and PCR reaction.
Step 5: After the amplification reaction is completed, rotate again to move the adsorbent to the product detection chamber. The amplification product on the adsorbent is absorbed by the detection test strip through the communication hole. The result is observed with the naked eye through the observation window on the test strip groove.

A detection device equipped with the chip comprises a graphene heating component, a power source, and a power control module. The graphene heating component is connected to the power source, and the power source is controlled by the power control module. The power control module controls the power source to perform on/off cycling operations on the graphene heating component. The graphene heating component is in contact with the disc-type microfluidic chip for nucleic acid detection. When powered on, it heats the disc-type microfluidic chip for nucleic acid detection; when powered off, it allows the disc-type microfluidic chip for nucleic acid detection to cool, thereby achieving a heating/cooling cycle for the disc-type microfluidic chip for nucleic acid detection.

Compared with the existing technology, the laser-induced graphene PCR detection device of the present invention has the following advantages:
1. The present invention provides a novel heating and cooling chip based on laser-induced graphene. The chip is prepared by laser induction, requiring no mask. The preparation pattern can be freely designed according to actual needs, with controllable morphology and adjustable composition. The preparation method is simple, allows for batch production, and is low-cost. It can effectively reduce the cost of thermal cycling equipment in current PCR instruments and is suitable for rapid on-site testing scenarios.
2. The laser-induced graphene material prepared by the present invention has good mechanical robustness and thermal stability, excellent electrical and thermal conductivity. It can rapidly generate and conduct heat through the Joule heating effect. At the same time, it possesses good porosity, facilitating heat dissipation and cooling. Therefore, the laser-induced graphene heating and cooling chip can achieve ultra-fast heating and cooling simply by controlling the voltage. Applying a low voltage enables ultra-fast heating (>10 °C/s); upon shutting off the voltage, it relies on its good porosity to autonomously achieve ultra-fast cooling (>10 °C/s) without the need for other auxiliary cooling methods. This reduces the size of the amplification chip and equipment, effectively applying it in the field of point-of-care rapid detection.

Compared with the existing technology, the disc-type microfluidic chip for nucleic acid detection of the present invention has the following advantages:
1. It features a high degree of integration and low consumption of reagents and samples. It only requires a drop of blood or other bodily fluid from the test subject to achieve "sample in, result out".
2. It is small in size, easy to carry, and requires no instruments or equipment. It only needs a finger to turn the handle, making it easy to operate and placing low technical demands on the operator.
3. With the aid of an immunostrip, test results can be read manually within just a few minutes. Result interpretation is intuitive and requires no instruments.
4. It is low-cost, easy to process, and suitable for mass production.

### Description of the Drawings

To illustrate the specific embodiments of the present invention or the technical solutions in the prior art more clearly, the following briefly introduces the drawings required for the description of the specific embodiments or the prior art. Obviously, the drawings in the following description are some embodiments of the present invention. For those of ordinary skill in the art, other drawings can be obtained based on these drawings without inventive effort.
Figure 1 shows the XPS C1s peak fitting of the graphene material prepared by the method of the present invention (laser fluence of 36 J/cm²).
Figure 2 shows surface morphology images of graphene induced by lasers with fluences of 5.5 J/cm², 5.8 J/cm², 10 J/cm², 15 J/cm², 20 J/cm², 25 J/cm², 30 J/cm², 36 J/cm², 38 J/cm², 40 J/cm², 46 J/cm², 50 J/cm², 60 J/cm², 70 J/cm², and 80 J/cm² (Nos. 1-15).
Figure 3 is a voltage-temperature variation graph of the graphene material induced by a laser with a fluence of 40 J/cm².
Figure 4 is a voltage-temperature variation graph of the graphene material induced by a laser with a fluence of 36 J/cm².
Figure 5 is a graph showing the relationship between the maximum temperature and heating rate on the laser-induced graphene surface and the input power.
Figure 6 is a comparison graph of the surface temperature of laser-induced graphene under the same input power.
Figure 7 is a graph showing the change in surface temperature of laser-induced graphene when the applied voltage is increased in steps.
Figure 8 is a comparison graph of surface temperatures under the same applied voltage for initial heating and stepwise heating.
Figure 9 is a comparison graph of the cooling rate of laser-induced graphene versus other materials.
Figure 10 is a temperature cycle graph of laser-induced graphene (35°C - 120°C).
Figure 11 is a graph showing the temperature change of the laser-induced graphene heat source and the heated liquid.
Figure 12 is a schematic diagram of the preparation process of the laser-induced graphene heating and cooling chip of the present invention.
Figure 13 is a schematic diagram of the connection of the laser-induced graphene heating and cooling chip.
Figure 14 is a schematic diagram of the detection process using the laser-induced graphene heating and cooling chip.
Figure 15 is a graph showing the measured heating and cooling performance of the laser-induced graphene heating and cooling chip.
Figure 16 is a comparison of the heat conduction path between the laser-induced graphene heating and cooling chip of the present invention and a traditional PCR instrument.
Figure 17 is a structural schematic diagram of the PCR reaction chamber.
Figure 18 is a structural schematic diagram of the disc-type microfluidic chip for nucleic acid detection.
Figure 19 is a structural schematic diagram of the upper surface of the top cover layer.
Figure 20 is a structural schematic diagram of the lower surface of the top cover layer.
Figure 21 is a structural schematic diagram of the reservoir layer.
Figure 22 is a schematic diagram of the principle of the immunostrip.
Figure 23 is a schematic diagram of the immunostrip detection results.

In the figures: 1 - Laser; 2 - Laser-induced graphene heating and cooling chip; 3 - Base material layer; 4 - Power source; 5 - Power control module; 6 - PCR reaction chamber; 7 - Sample introduction layer; 8 - Detection layer; 9 - Test strip; 10 - Sealing plug; 11 - Rotating column; 12 - Injection port; 13 - Circular slide rail; 14 - Sample introduction chamber; 15 - Front washing chamber; 16 - Rear washing chamber; 17 - Amplification reaction chamber; 18 - Detection chamber; 19 - Observation window; 20 - Test strip groove;
21 - Top cover layer; 22 - Reservoir layer; 23 - Test strip; 24 - Lid; 25 - Rotating handle; 26 - Sample inlet; 27 - First outer slide track; 28 - First inner slide track; 29 - Nucleic acid filter paper fixation hole; 30 - Filter paper; 31 - Second outer slide track; 32 - Second inner slide track; 33 - Sample addition well; 34 - First washing well; 35 - Second washing well; 36 - Amplification reaction well; 37 - Product detection well; 38 - Test strip insertion port; 39 - Observation window; 40 - Test strip groove; 41 - Sample pad; 42 - Nitrocellulose membrane; 43 - Conjugate pad; 44 - Absorbent pad; 45 - Backing card; 46 - Test line; 47 - Control line.

### Detailed Description

The technical solutions in the embodiments of the present application will be described clearly and completely below in conjunction with the accompanying drawings in the embodiments of the present application. Obviously, the described embodiments are merely illustrative and are not intended to limit the present application.

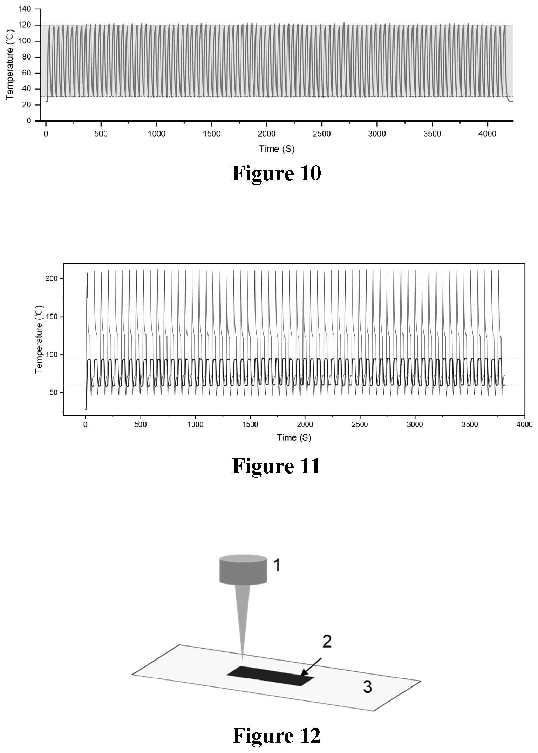

The present invention first discloses a method for preparing graphene using laser induction as a heating device for PCR detection, and the resulting graphene sheet material. As shown in Figure 12, the method comprises: reducing a precursor material layer using laser-induced processing.

In one embodiment of the present invention, the method for preparing laser-induced graphene includes: providing a carbon-containing precursor material layer, and using a laser to scan the surface of the carbon-containing precursor material layer. The localized heat generated by the laser irradiation causes the surface of the precursor material to carbonize, thereby forming graphene.

Specifically, during the preparation process, a special carbon-containing precursor material layer is provided. This precursor material layer can be located on the base material layer 3. The precursor material layer and the base material layer can be an integrated single material or separate, different materials. Precursor materials include, but are not limited to, polyimide, phenolic resin, polyethyleneimine, lignocellulose, wood, etc. A laser 1 is used to directly scan the surface of the aforementioned special carbon-containing precursor material layer. The localized heat generated by the laser irradiation causes the surface of the precursor material to carbonize. During the carbonization process, carbon atoms are recombined from sp3 bonds to sp2 bonds, and the oxygen and nitrogen groups in the precursor material are decomposed, thereby obtaining the laser-induced graphene heating and cooling chip 2. Lasers used include, but are not limited to, carbon dioxide lasers, femtosecond lasers, etc.

To meet the requirements for rapid heating and cooling in PCR detection devices, the initial laser fluence for forming the laser-induced graphene material in this embodiment is 5.5 J/cm². By adjusting the laser parameters, the physical and chemical properties of the laser-induced graphene material are controlled. Increasing the laser power can increase the thickness of the laser-induced graphene material, enhance its electrical conductivity, and consequently reduce its resistance. According to the Joule heating effect (Q = V²/R * t), under the same input voltage conditions, the smaller the resistance, the more heat is generated within the same time period, and the faster the heating rate. Simultaneously, increasing the laser power during the preparation process will increase the release rate of gases, thereby increasing the number of pores and enhancing the porosity and pore size of the laser-induced graphene material, achieving a faster cooling rate. However, exceeding a threshold laser power can lead to the destruction of the laser-induced graphene material structure and loss of functionality. Therefore, through design and actual validation, this embodiment optimizes the initial laser fluence for forming the laser-induced graphene material. Selecting a laser fluence of 5.5 J/cm² allows for obtaining a complete graphene structure while also achieving a higher number of pores and greater porosity, thus improving heat transfer efficiency and resulting in better heating and cooling performance.

It was surprisingly found that when the laser fluence for laser-induced processing is greater than or equal to 5.5 J/cm², preferably greater than 5.5 J/cm², more preferably 5.8-80 J/cm², even more preferably 5.8-60 J/cm², and most preferably 5.8-50 J/cm², LIG can be stably induced. Therefore, in one embodiment of the present invention, the laser fluence for laser-induced processing is greater than or equal to 5.5 J/cm², preferably greater than 5.5 J/cm², more preferably 5.8-80 J/cm², even more preferably 5.8-60 J/cm², and most preferably 5.8-50 J/cm². It was even more surprisingly found that when the laser fluence gradually increases from 0, it is difficult to form stable and uniform graphene on the polyimide base material when the fluence is less than 5.5 J/cm². When the fluence reaches 5.5 J/cm², graphene material can begin to be stably induced on the polyimide base material. As the fluence continues to increase beyond 5.5 J/cm², the graphitization degree of the laser-induced graphene material formed on the surface becomes more complete. Within the fluence range of 5.8-80 J/cm², especially 5.8-50 J/cm², the formation quality of laser-induced graphene gradually improves, the surface resistance gradually decreases, and the heating rate gradually increases. However, when the laser fluence exceeds 50 J/cm², the laser-induced graphene formed on the polyimide base material becomes difficult to adhere effectively, is prone to peeling off under slight external force, and exhibits reduced mechanical strength and increased brittleness.

To meet the requirements for rapid heating and cooling in PCR detection devices, the heating and cooling chip in the present invention adopts a sheet-like laser-induced graphene structure. By controlling the power, the laser-induced graphene material is concentrated on the surface layer of the base precursor material layer, with a thickness generally ranging from 1 to 120 µm, preferably 2-100 µm, 3-80 µm, or 5-60 µm.

The laser-induced graphene of the present invention is prepared by laser scanning on a polymer-based material. Compared with traditional graphene materials, it features a simple preparation process and high product performance reliability.

Laser-induced graphene possesses an extremely high electro-thermal conversion efficiency (>99%). Under low input power conditions, laser-induced graphene can rapidly reach a high temperature. Under operating conditions with an applied voltage of 8V and input power less than 2.4W, the maximum surface temperature can approach 300°C, and the maximum heating rate can reach 68.5°C/s. Compared with other electrothermal materials, it has significant advantages in both maximum temperature and heating rate. Furthermore, there is a clear functional relationship between the maximum surface temperature and heating rate of laser-induced graphene and the input electrical power. Under the same input power, independent laser-induced graphene can achieve the same surface temperature. This indicates that the temperature of laser-induced graphene can be conveniently and rapidly controlled by changing the input electrical energy density. Due to the extremely high electro-thermal conversion efficiency, as well as the material's inherent low specific heat capacity and multilayer porous structure, the rate of conversion from electrical energy to thermal energy is very fast. This also makes the temperature control of laser-induced graphene not only convenient but also rapid. Moreover, laser-induced graphene itself has a stable ability to regulate temperature through electrical power control.

Laser-induced graphene also possesses a rapid cooling capability. Due to its low input power and surface structure characterized by a loose, porous morphology (a unique microstructural advantage of laser-induced graphene compared to graphene formed by traditional chemical deposition or surface growth), heat can be dissipated quickly after power supply is cut off. The maximum natural cooling rate reaches 36°C/s, far exceeding that of traditional electrothermal materials.

Laser-induced graphene maintains stable performance during prolonged heating and cooling cycles. Experiments have shown that over 100 heating and cooling cycles, the heating/cooling rates and the achievable temperature levels remain stable in each cycle.

Furthermore, graphene exhibits very low thermal contact resistance with various materials (such as silicon dioxide, polymers like PMMA, polyethylene, etc.). This characteristic facilitates rapid and efficient heat transfer from the graphene to external objects. Through direct hard contact, it can effectively heat and cool the target object.

In summary, by leveraging the efficient electro-thermal conversion capability of laser-induced graphene, its fast and precise temperature response under applied voltage, rapid temperature change capability, and efficient heat transfer properties, precise regulation of liquid temperature can be achieved, meeting the stringent temperature requirements of the PCR process.

Therefore, the laser-induced graphene of the present invention is well-suited for application in PCR detection.

Consequently, in another aspect, the present invention also discloses the use of said laser-induced graphene in PCR detection.

### Laser-Induced Graphene PCR Detection Device

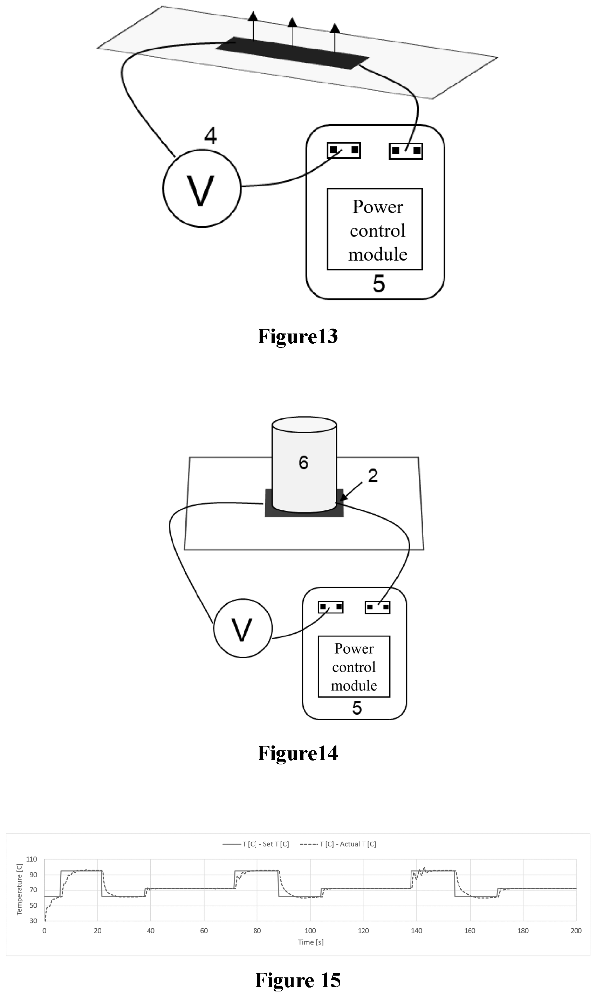

The present invention also discloses a PCR detection device prepared using the graphene material obtained as described above. As shown in Figures 13-14, it comprises a PCR reaction chamber 6, a graphene heating component, a low-voltage DC power source 4, and a power control module 5. The graphene heating component is the laser-induced graphene heating and cooling chip 2 obtained previously. The graphene heating component is placed in close contact with the PCR reaction chamber. When the low-voltage DC power source 4 supplies power to the graphene heating component, ultra-fast heating (>10°C/s) is achieved. After the voltage is turned off, ultra-fast cooling (>10°C/s) is autonomously achieved by relying on heat dissipation to the surrounding environment, without the need for other auxiliary cooling methods. The measured heating and cooling performance is shown in Figure 15, where the solid line represents the set temperature and the dotted line represents the actual temperature. It can be seen from the figure that the actual temperature closely matches the set temperature. Furthermore, the means of supplying voltage include, but are not limited to, batteries, rechargeable batteries, portable power banks, mobile phones, power supply units, and 220V mains power.

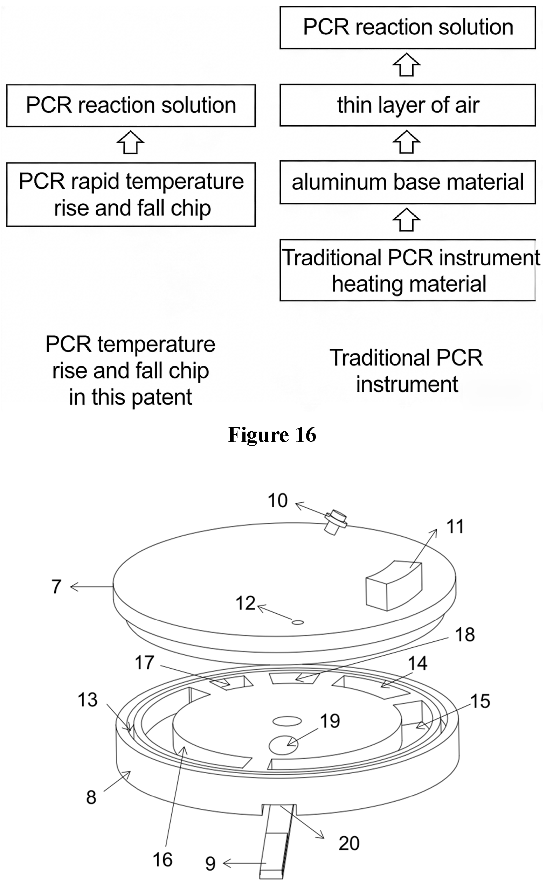

The volume of the aforementioned PCR reaction micro-chamber is 20-100 µL, preferably 25-100 µL, and can accommodate fluorescent quantitative PCR reaction reagents (typically 20 µL, including enzymes, substrates, primers, fluorescent molecules, and the target detection substance). As shown in Figure 16, in the present invention, the graphene heating and cooling chip is placed in close contact with the PCR reaction chamber. Heat is conducted directly from the graphene heating and cooling chip to the PCR reaction chamber. In contrast to traditional PCR instruments, where heat conduction needs to travel from the traditional PCR instrument's heating material to an aluminum base plate, then through a thin layer of air, and finally to the PCR reaction chamber, the present invention offers high heat conduction efficiency, low heat loss, and fast heating and cooling rates. It reduces the multiple steps in the heat conduction path found in traditional PCR instruments, effectively improving heat conduction efficiency and further enhancing the reaction speed.

The present invention also provides a control method for the aforementioned detection. Specifically, the control method includes controlling the power supply's on/off time (or power supply voltage) to the graphene heating component during the thermal cycling process. This on/off time (or power supply voltage) control is implemented by the power control module. Precise control of heating and cooling under different conditions (based on environmental factors such as temperature, wind speed, etc.) is achieved, with cycles ranging from 1 to 120 times, preferably 1-80 times, more preferably 1-50 times, for example, 45 times.

As described above, the present invention provides a novel heating and cooling chip based on laser-induced graphene, capable of autonomously achieving ultra-fast heating and cooling. This chip is prepared using laser-induced processing, allowing for batch production and low cost. It can improve the temperature control module of current PCR instruments, reduce costs, and increase PCR reaction speed. Furthermore, this laser-induced graphene-based heating and cooling chip can be effectively applied in the field of point-of-care rapid detection, thereby promoting the true realization of on-site rapid detection using PCR technology. It can also be used in high-throughput benchtop detection devices.

Optionally, the PCR reaction chamber is a disc-type PCR detection chip with a two-layer structure, comprising from top to bottom: a sample introduction layer 7 and a detection layer 8.

As shown in Figure 17, the sample introduction layer 7 of the present invention has a diameter of 60 mm and a height of 3 mm. Its upper surface is provided with a rotating column 11 protruding 5 mm in height, which can be pushed with a finger to rotate the sample introduction layer. On one side of the rotating column 11, there is an injection port 12 with a diameter of 4 mm and a height of 2 mm, and a sealing plug 10 matching the size of the injection port 12, used to seal the injection port after sample addition to prevent contamination.

The detection layer 8 has a diameter of 60 mm and a height of 7 mm. A circular slide rail 13 is provided on the outer side of the upper surface of the detection layer 8. Correspondingly, the lower surface of the sample introduction layer is provided with a circular slider. This slide rail and slider arrangement allows the sample introduction layer to rotate within the detection layer. When the sample introduction layer rotates, the cellulose filter paper within it can enter different reaction chambers through rotation.

A filter paper fixing hole is located below the injection port 12 for embedding cellulose filter paper. The cellulose filter paper is cut into a circular shape with a diameter of 6mm, pasted to the lower part of the injection port with 3M tape, and can completely cover the injection port. This allows the liquid to be directly absorbed by the cellulose filter paper after sample injection. When the sample is adsorbed onto the cellulose filter paper, the negatively charged macromolecular DNA curls and binds tightly to the cellulose filter paper matrix.

Each reaction chamber is cut out in the form of a circular ring with an outer diameter of 50mm and an inner diameter of 40mm on the detection layer 8. These chambers are arranged in sequence, including a sample injection chamber 14, a pre-washing chamber 15, a post-washing chamber 16, an amplification reaction chamber 17, and a detection chamber 18. The reaction chambers are independent of each other to prevent liquid leakage. A strip sample inlet hole is set up in the detection chamber and sealed with paraffin.

The front washing chamber 15 and the rear washing chamber 16 accommodate 500µL of washing buffer, such as Tris buffer, TE buffer, FTA card purification reagent, etc. The amplification reaction chamber 17 holds 50µL of reaction buffer, including RPA reagent, LAMP reagent, etc. The detection chamber 18 contains 100µL of running buffer, such as Tris buffer, TE buffer, etc. The sample inlet hole of the test strip is sealed with paraffin. Paraffin with a melting point around 60°C is selected. After the amplification reaction is completed, the cellulose filter paper is rotated to the detection chamber, allowing the product in the cellulose filter paper 10 to mix with the reaction buffer in the detection chamber 18. Then, the temperature of the heating device at this location is raised to around 60°C to melt the paraffin, allowing the liquid to fall onto the test strip 9. The test strip groove 20 is located on the back of the detection layer 8 and is connected to the sample inlet hole of the test strip. The observation window 19 is located in the test strip groove and is an oval through-hole, facilitating visual inspection of the results of the immunochromatographic test strip.

The sample introduction layer and detection layer underwent surface hydrophobic treatment. This involved immersing the two-layer structure in a trichloro (1H,1H,2H,2H-perfluorooctyl) silane reagent for three hours in vacuum. This allowed the silane to form a self-assembled monolayer on the inorganic surface, enhancing its hydrophobicity and preventing liquid leakage.

The PCR reaction chamber is constructed by fastening its upper and lower layers together, forming a sealed space to prevent liquid leakage. On the front side, according to the sample inlet chamber, front washing chamber, rear washing chamber, and amplification reaction chamber of the detection layer, numbers such as ①, ②, ③, and ④ are marked. Imitating the appearance of a rotary dial telephone, by rotating, the cellulose filter paper can enter different reaction chambers to achieve the three processes of sample introduction, washing, and amplification, respectively.

When conducting nucleic acid detection using PCR, this invention employs cellulose filter paper as the adsorption material for nucleic acid detection. It can quickly bind to nucleic acids from complex biological samples, retain them after a simple washing step, and directly release them into the amplification reaction system. In this invention, the ability of different cellulose filter papers to bind to nucleic acids in blood was explored. It was found that FTA cards can rapidly capture nucleic acids from body fluids such as saliva, urine, blood, and serum. Therefore, utilizing a rapid and cost-effective method based on cellulose filter paper for purifying nucleic acids from different biological samples provides a key technical means for the PCR detection chip of this invention to perform rapid and instantaneous detection in resource-limited environments.

After nucleic acid extraction, when detecting the nucleic acid amplification products, an immunochromatographic test strip is used for the detection of the post-amplification products. The immunochromatographic test strip is loaded into the test strip groove, and the sample pad is placed below the sample inlet of the test strip. The detection line and quality control line are located below the observation window in the test strip groove.

Taking the detection of EGFR gene in blood by nucleic acid isothermal amplification as an example, cellulose filter paper and immuno-test strips are applied to the PCR detection chip of this invention. 500 µL of Tris buffer is added to the pre-washing chamber and post-washing chamber, respectively; 50 µL of loop-mediated isothermal amplification reaction buffer is added to the amplification reaction chamber, and 100 µL of TE buffer is added to the detection chamber as the running buffer. EGFR plasmids of certain concentrations are added to normal human blood, with final concentrations of 10⁵ copies/mL, 10⁴ copies/mL, 10³ copies/mL, and 10² copies/mL. 15 µL of this series of samples is injected into the injection port of the PCR detection chip of this invention, allowing the nucleic acid plasmids to adsorb onto the cellulose filter paper. After two washes, the cellulose filter paper is placed in the nucleic acid amplification reaction buffer, and the heating device is turned on to raise the temperature of the amplification reaction chamber to 65°C for nucleic acid amplification. The reaction is completed after 40 minutes. The cellulose filter paper is rotated to the detection chamber, and the amplification products on the cellulose filter paper are diluted with the running buffer. The temperature of the reaction chamber is then raised to 80°C to melt the paraffin in the detection chamber, and the products are absorbed by capillary force through the sample injection hole of the test strip onto the immuno-test strips for color development reaction. The reaction results can be visually interpreted through the observation window. The immuno-test strips ①, ②, ③, ④, and ⑤ correspond to 10⁵ copies/mL, 10⁴ copies/mL, 10³ copies/mL, 10² copies/mL, and a blank control, respectively. Among them, immuno-test strips ①, ②, and ③ all show red bands on the detection and quality control lines, while immuno-test strips ④ and ⑤ only show red bands on the quality control line, indicating that the PCR detection chip of this invention can achieve a detection limit of 103 copies/mL.

Therefore, the present invention designs a low-cost, portable chip for rapid on-site nucleic acid detection by PCR. Through simple rotational operations, it can meet the demand for rapid, on-site nucleic acid detection, especially in resource-limited regions. Its advantages are as follows:
(1) Inspired by the appearance of a rotary dial telephone, a disc-type PCR detection chip is designed. The entire process, including nucleic acid extraction, washing and amplification, can be achieved simply by rotating the column with a finger, resulting in low technical requirements for operators. The chip features high integration and low consumption of reagents and samples. Only a drop of blood or other body fluid from the subject is needed to realize "sample-in, result-out" detection. Finally, the results can be read manually with an immunochromatographic test strip without any equipment.
(2) The disc-type PCR detection chip is easy to fabricate and low in cost, including the preparation of the PCR chip, as well as the nucleic acid amplification reaction buffer, enzymes, primers and other components.
(3) The total detection time is less than 60 minutes, including nucleic acid extraction and amplification, which is favorable for rapid point-of-care screening of pathogens.

A nucleic acid detection method based on a disc-type PCR detection chip comprises the following five steps:
Step 1: Rotate the injection port of the sample layer to above the sample injection chamber of the detection layer for sample introduction. A certain volume of body fluid, such as blood, serum, urine, saliva, etc., is injected onto the surface of the cellulose filter paper through the injection port using a pipette. Wait for 3-5 minutes until the liquid dries. Special chemicals in the cellulose filter paper can autonomously lyse cells in the body fluid and bind to negatively charged nucleic acids.
Step 2: Toggle the rotation column of the sample layer with a finger to rotate the cellulose filter paper to the front washing chamber. Wait for 2 minutes to wash away impurities on the surface of the cellulose filter paper, such as cells, proteins, phenols, etc. Slightly toggling the rotation column left and right can accelerate the washing speed.
Step 3: Toggle the rotation column of the sample layer again to rotate the cellulose filter paper to the rear washing chamber. Wait for another 2 minutes to achieve a more thorough washing. Slightly toggling the rotation column left and right can accelerate the washing speed.
Step 4: Toggle the rotation column of the sample layer to rotate the cellulose filter paper to the amplification reaction chamber. Then turn on the heating device. When the temperature rises to a certain level, the reaction buffer in the amplification reaction chamber will undergo an amplification reaction with the nucleic acids on the cellulose filter paper, including but not limited to isothermal amplification and PCR.
Step 5: After the reaction is completed, rotate again to move the cellulose filter paper to the detection chamber. The amplification product on the cellulose filter paper is absorbed by the sample pad of the immunochromatographic test strip through the communication hole, and the result is displayed on the test line and control line. The result can be observed with the naked eye through the observation window on the test strip groove.

In summary, the above device is proven to have significant social and economic benefits for expanding molecular detection of clinical pathogens, epidemiological investigation, food inspection and quarantine, and other fields in resource-limited settings.

### Disc-Type Microfluidic Chip for Nucleic Acid Detection

The technical solutions in the embodiments of the disc-type microfluidic chip for nucleic acid detection of the present application will be described clearly and completely below with reference to the accompanying drawings in the embodiments of the present application. Obviously, the described embodiments are only for illustration, not for limiting the present application.

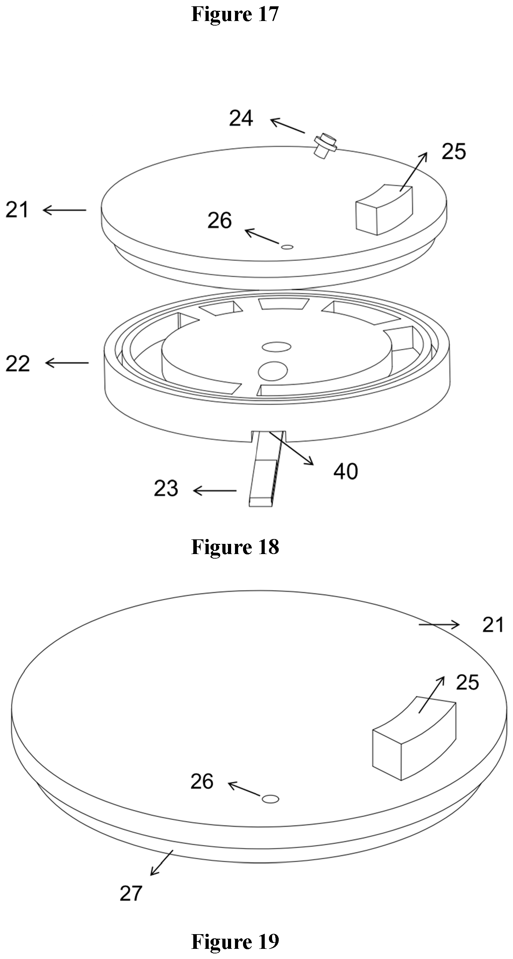

As shown in Figure 18, the disc-type microfluidic chip for nucleic acid detection of the present invention has a two-layer structure, which includes, from top to bottom: a top cover layer 21 and a reservoir layer 22.

The top cover layer 21 of the present invention has a diameter of 60 mm and a height of 3 mm. A rotating handle 25 with a protrusion height of 5 mm is arranged on the upper surface, which can be pushed by a finger to rotate the top cover layer. On one side of the rotating handle 25, there is a sample inlet 26 with a diameter of 4 mm and a height of 2 mm, and a cover 24 matching the size of the sample inlet 26, which is used to seal the sample inlet after sample addition to prevent contamination, as shown in Figure 19.

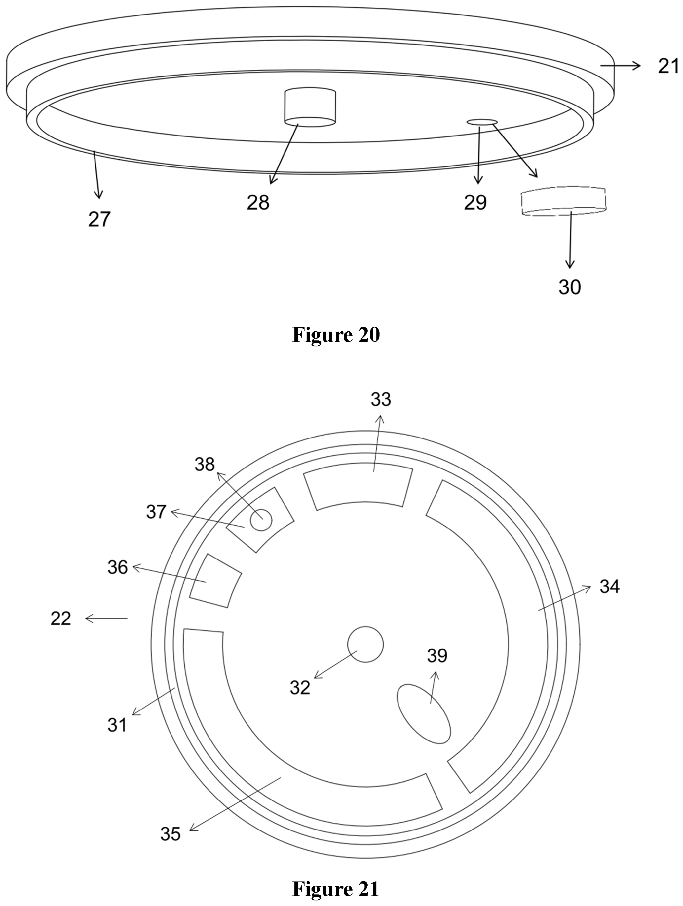

Below the top cover layer 21 are designed an annular first outer slide 27 and a first inner slide 28, which are used to facilitate the rotation of the top cover layer in the reservoir layer. The first outer slide 27 has an inner diameter of 54 mm, an outer diameter of 56 mm, and a height of 3 mm. The first inner slide 28 has a diameter of 5 mm and a height of 3 mm. Below the sample inlet 26, there is a nucleic acid filter paper fixing hole 29 with a diameter of 6 mm and a height of 1 mm for embedding cellulose filter paper 30. The cellulose filter paper 30 is cut into a circle with a diameter of 6 mm, adhered under the sample inlet with 3M tape, and can completely cover the sample inlet so that the liquid is directly absorbed by the cellulose filter paper after sample injection, as shown in Figure 20. When the sample is adsorbed onto the cellulose filter paper, the negatively charged macromolecular DNA is coiled and tightly bound to the cellulose filter paper matrix.

As shown in Figure 21, the reservoir layer 22 has a diameter of 60 mm and a height of 7 mm. An annular second outer slide 31 with a depth of 3 mm is formed on the upper surface of the reservoir layer in the form of a ring with an outer diameter of 56 mm and an inner diameter of 54 mm, and a cylindrical second inner slide 32 is formed as a groove with a diameter of 5 mm. They are highly compatible with the protruding annular first outer slide 27 and first inner slide 28 in the top cover layer 21, facilitating the rotation of the top cover layer. That is, when the top cover layer rotates, the cellulose filter paper in the top cover layer can be rotated into different reaction chambers.

Annular reaction chambers with a depth of 2.5 mm are formed on the reservoir layer in the form of a ring with an outer diameter of 50 mm and an inner diameter of 40 mm. They sequentially include a sample addition chamber 33, a first washing chamber 34, a second washing chamber 35, an amplification reaction chamber 36, and a product detection chamber 37. The reaction chambers are independent of each other to prevent cross-leakage. A test strip injection hole 38 is arranged in the product detection chamber and sealed with paraffin.

The bottom area of the sample pool 33 is 68mm²; the bottom areas of both the first washing pool 34 and the second washing pool 35 are 235mm², accommodating 500µL of washing buffer, such as Tris buffer, TE buffer, FTA card purification reagent, etc.; the bottom area of the amplification reaction pool 36 is 30mm², accommodating 50µL of reaction buffer, including RPA reagent, LAMP reagent, etc.; the bottom area of the product detection pool 37 is 40mm², accommodating 100µL of running buffer, such as Tris buffer, TE buffer, etc. The test strip sample inlet 38 is a through-hole with a diameter of 3mm, sealed with paraffin. Paraffin with a melting point around 60°C is selected. After the amplification reaction is completed, the cellulose filter paper is rotated to the product detection pool, allowing the product in the cellulose filter paper 30 to mix with the reaction buffer in the product detection pool 37. Then, the temperature of the heating device at this location is raised to around 60°C, melting the paraffin and allowing the liquid to fall onto the test strip 23; the test strip groove 40 is located on the back of the reservoir layer 22, with a length of 60mm, width of 6mm, and height of 2mm, communicating with the test strip sample inlet; the observation window 39 is located in the test strip groove, being an elliptical through-hole with a major axis of 10mm and a minor axis of 5mm, facilitating visual inspection of the results of the immunochromatographic test strip.

The upper and lower layers of the device are constructed using materials with low cost, easy processing, and good biocompatibility, such as PMMA, epoxy resin, PVC, PC, etc., as substrates. Channels and reaction tanks are directly fabricated using laser etching technology, or structures are etched on a PMMA substrate, followed by mold-casting with PDMS. The top cover layer and the reservoir layer undergo surface hydrophobic treatment, which involves immersing the two-layer structure in trichloro (1H,1H,2H,2H-perfluorooctyl) silane reagent in vacuum for three hours, allowing the silane to form a self-assembled monolayer on the surface, thereby increasing its hydrophobicity and preventing liquid leakage.

The upper and lower layers of the chip are buckled together, forming a sealed space as a whole to prevent liquid leakage. The front side can be marked with numbers 1, 2, 3, and 4 according to the sample pool, the first washing pool, the second washing pool, and the amplification reaction pool of the reservoir layer. Imitating the appearance of a rotary dial telephone, by rotating, the cellulose filter paper can enter different reaction pools to achieve the three processes of sample introduction, washing, and amplification respectively.

This invention utilizes cellulose filter paper as a nucleic acid adsorption material, which can rapidly bind to nucleic acids from complex biological samples. After a simple washing step, the nucleic acids are retained and can be directly released into the amplification reaction system. In this invention, the ability of different materials of cellulose filter paper to bind to nucleic acids in blood was explored. 15µL of blood was collected from a lung cancer patient and dropped onto different materials of cellulose filter paper, respectively. After drying for 3-5 minutes, they were soaked and washed twice in 0.1M Tris buffer for 2-3 minutes each time. Finally, they were placed in LAMP reaction buffer for amplification for 40 minutes, and EGFR gene detection was performed. The amplification products were detected by agarose gel electrophoresis. A4 paper, toilet paper, FTA cards, hand wipes, and reagent kits were used, respectively. The results showed that, except for A4 paper, the nucleic acids in blood were rapidly captured by the cellulose in toilet paper, FTA cards, and hand wipes and retained during the washing step. The capture efficiency was consistent with the extraction method using a reagent kit, especially for FTA cards, which were soaked in special denaturants and chelating agents to adsorb more nucleic acids. When using FTA cards to adsorb different biological samples, it was found that FTA cards could rapidly capture nucleic acids from body fluids such as saliva 1, urine 2, blood 3, and serum 4. Therefore, the rapid and economical method of purifying nucleic acids from different biological samples based on cellulose filter paper provides a key technical means for the rapid and instant detection of the microfluidic chip of this invention in resource-limited environments.

After nucleic acid extraction, when detecting the nucleic acid amplification products, an immunochromatographic test strip is used for the detection of the post-amplification products. The immunochromatographic test strip is characterized by low cost, rapid simplicity, and intuitive result interpretation. The immunochromatographic test strip is loaded into the test strip groove, and the sample pad is placed below the sample inlet of the test strip. The detection line and quality control line are located below the observation window in the test strip groove.

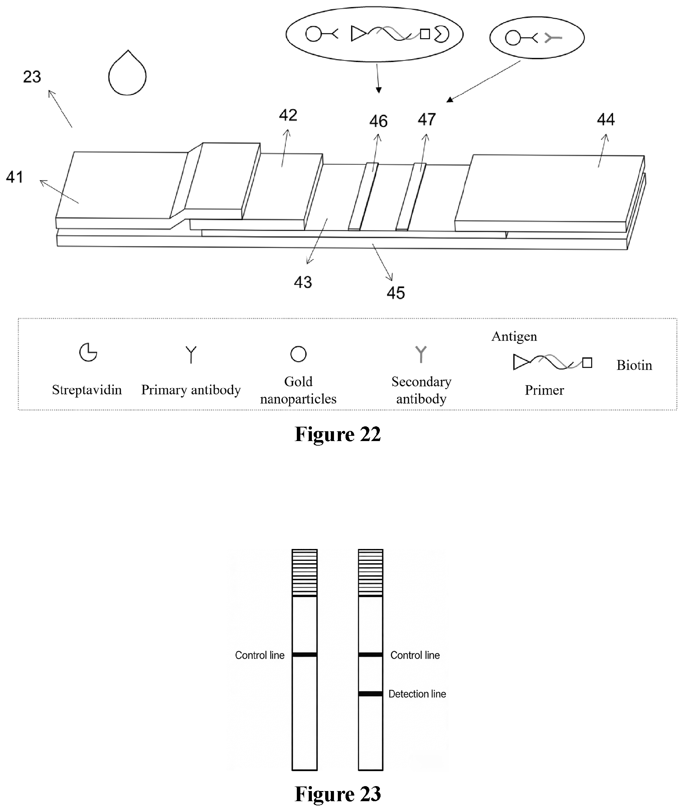

The immunochromatographic test strip is a method that utilizes the principle of specific binding between antibodies and antigens to detect target substances in samples. Its basic structure is shown in Figure 22. On the base plate 45, the sample pad 41, gold conjugate pad 43, nitrocellulose membrane 42, and absorbent pad 44 are sequentially attached. The gold conjugate pad contains a primary antibody labeled with colloidal gold. The nitrocellulose membrane is coated with streptavidin on the detection line and with a secondary antibody (goat anti-mouse IgG) on the quality control line. The forward primer 5 is modified with biotin at the end, while the reverse primer 5 is modified with antigen at the end. Table 1 provides an example of primer sequences used to detect the EGFR gene in the blood of lung cancer patients.

During testing, when a sample containing the substance to be detected is added to the sample pad, under the effect of chromatography, the sample moves towards the absorbent pad, first passing through the gold conjugate pad, where it specifically binds to the gold conjugate antibody, and then continues to move towards the absorbent paper end. When the sample reaches the detection line 46, the substance to be detected (which has bound to the gold conjugate antibody) specifically binds to the streptavidin in the detection line in a double sandwich manner, resulting in the display of a red band. The gold conjugate antibody that has not bound to the substance to be detected continues to chromatograph and moves to the quality control line 47, where it specifically binds to the secondary antibody, resulting in the display of a red band, as shown in Figure 23.

Taking the detection of EGFR gene in blood by nucleic acid isothermal amplification as an example, cellulose filter paper and immuno-test strips are applied to the microfluidic chip of this invention. 500µL of Tris buffer solution is added to each of the first and second washing pools, 50µL of loop-mediated isothermal amplification reaction buffer solution is added to the amplification reaction pool, and 100µL of TE buffer solution is added to the product detection pool as the running buffer. EGFR plasmids of certain concentrations are added to normal human blood, with final concentrations reaching 10⁵ copies/mL, 10⁴ copies/mL, 10³ copies/mL, and 10² copies/mL, respectively. 15µL of this series of samples is injected into the sample inlet of the microfluidic chip of this invention, allowing the nucleic acid plasmids to adsorb onto the cellulose filter paper. After two washes, the cellulose filter paper is placed in the nucleic acid amplification reaction buffer solution, and the heating device is turned on to raise the temperature of the amplification reaction pool to 65°C for nucleic acid amplification. The reaction is completed after 40 minutes. The cellulose filter paper is rotated to the product detection pool, where the amplification products on the filter paper are diluted with the running buffer, and the temperature of the reaction pool is raised to 80°C to melt the paraffin in the product detection pool. The products are then absorbed by capillary force through the sample inlet of the test strip onto the immuno-test strip for color development reaction. The reaction results can be visually interpreted through the observation window. Immuno-test strips 1, 2, 3, 4, and 5 correspond to 10⁵ copies/mL, 10⁴ copies/mL, 10³ copies/mL, 10² copies/mL, and a blank control, respectively. Among them, immuno-test strips 1, 2, and 3 all show red bands on the detection and quality control lines, while immuno-test strips 4 and 5 only show red bands on the quality control line, indicating that the microfluidic chip of this invention can achieve a detection limit of 10³ copies/mL.

Therefore, the present invention provides a low-cost, portable chip for rapid on-site nucleic acid detection. Through simple rotational operations, it meets the demand for rapid, on-site nucleic acid detection, especially in resource-limited regions. Its advantages are as follows:

Inspired by the appearance of a rotary dial telephone, a disc-type microfluidic chip is designed. The entire process, including nucleic acid extraction, washing and amplification, can be accomplished simply by turning the handle with a finger, resulting in low technical requirements for operators.

The chip features high integration and low consumption of reagents and samples. Only a drop of blood or other body fluid from the subject is needed to achieve "sample-in, result-out" detection. Finally, the results can be read manually with an immunochromatographic test strip without the need for any equipment.

(3) The disc-type microfluidic chip is easy to manufacture and low in cost, covering all components, including chip fabrication, nucleic acid amplification buffer, enzymes, and primers.

(4) The total detection time is less than 60 minutes, including nucleic acid extraction and amplification, which is favorable for rapid point-of-care screening of pathogens.

An operational process for a disc-type microfluidic chip based on nucleic acid detection, comprising the following five steps:
Step 1: Rotate the sample inlet of the top cover layer above the sample dispensing pool of the reservoir layer and inject a certain amount of bodily fluids, such as blood, serum, urine, saliva, etc. into the surface of the cellulose filter paper through the sample inlet using a pipette. Wait for 3-5 minutes to allow the liquid to dry. The special chemical substances in the cellulose filter paper can autonomously lyse the cells in the bodily fluids and bind to negatively charged nucleic acids.
Step 2: Use your fingers to rotate the rotating handle of the top cover layer, moving the cellulose filter paper to the first washing tank. Wait for 2 minutes to wash away impurities on the surface of the cellulose filter paper, such as cells, proteins, phenols, etc. Gently moving the rotating handle side to side can speed up the washing process.
Step 3: Turn the rotating handle of the top cover layer again to move the cellulose filter paper to the second washing basin. Wait for another 2 minutes to ensure more thorough washing. Gently moving the rotating handle side to side can speed up the washing process.
Step 4: Rotate the rotating handle of the top cover layer to move the cellulose filter paper to the amplification reaction chamber. Then, turn on the heating device. When the temperature rises to a certain level, the reaction buffer in the amplification reaction chamber will undergo an amplification reaction with the nucleic acid on the cellulose filter paper, which is not limited to isothermal amplification reaction and PCR reaction.
Step 5: After the reaction is complete, rotate the cellulose filter paper again to move it to the product detection cell. The amplification products on the cellulose filter paper are absorbed by the sample pad on the immunochromatographic strip through the communication hole, and the results are displayed on the detection line and quality control line. The results can be observed visually through the observation window on the groove of the strip.

In summary, the proposed device demonstrates significant socio-economic benefits in expanding our capabilities in molecular detection of clinical pathogens, epidemiological investigations, food inspection and quarantine, and other fields in resource-limited environments.

**Table 1. Primer sequences used for detecting EGFR gene in the blood of lung cancer patients**

| Target gene | Primer sequence | Product size |
|---|---|---|
| | F: TCCAGGAAGCCTACGTGATG | |
| EGFR | | 109 bp |
| | R: CCGAAGGGCATGAGCTGCA | |

The present invention also discloses a detection device equipped with the aforementioned disc-type nucleic acid detection microfluidic chip, which includes a graphene heating device, a power supply, and a power supply control module. The graphene heating device is connected to the power supply, which is controlled by the power supply control module. The power supply control module controls the power supply to perform an on/off cycle operation on the graphene heating device. The graphene heating device is in contact with the disc-type nucleic acid detection microfluidic chip and heats the disc-type nucleic acid detection microfluidic chip when powered on, and cools it down when powered off, thereby achieving a temperature rise/drop cycle for the disc-type nucleic acid detection microfluidic chip.

The graphene heating device comprises a laser-induced graphene layer and a substrate material layer. The laser-induced graphene layer is located on the surface layer of the substrate material layer, with a thickness ranging from 1 to 120 µm, preferably from 2 to 100 µm, more preferably from 3 to 80 µm, or most preferably from 5 to 60 µm. During the preparation process, a special precursor material containing carbon is provided, which is placed on the material substrate. The precursor material includes, but is not limited to polyimide, phenolic resin, polyethyleneimine, lignocellulose, wood, etc. The surface of the aforementioned special precursor material containing carbon is directly scanned using a laser. The local heat generated by laser irradiation causes the surface of the precursor material to carbonize. During the carbonization process, carbon atoms are reorganized from sp³ bonds to sp² bonds, and the oxygen and nitrogen groups of the precursor material are decomposed, thereby obtaining the laser-induced graphene heating device.

To meet the rapid heating and cooling requirements of nucleic acid detection microfluidic chips, in this implementation, the laser fluence for the initial formation of laser-induced graphene material is set at 5.5 J/cm². By adjusting the laser parameters, the physical and chemical properties of the laser-induced graphene material are regulated. Increasing the laser power can enhance the thickness of the laser-induced graphene material, improve its electrical conductivity, and thereby reduce its resistance. According to the current thermal effect (Q = V²/R * t), under the same input voltage, a lower resistance results in more heat generated within the same time period, leading to faster heating rates. Additionally, increasing the laser power during the preparation process accelerates gas release, increasing the number of pores and enhancing the porosity and pore size of the laser-induced graphene material, thereby achieving faster cooling rates. However, if the laser power exceeds a certain threshold, it can damage the structure of the laser-induced graphene material, rendering it non-functional. In this implementation, the laser fluence for the initial formation of laser-induced graphene material is set at 5.5 J/cm².

The graphene heating device is tightly attached to the disc-type nucleic acid detection microfluidic chip. When a low-voltage DC power supply powers the graphene heating device, ultra-fast heating (>10°C/s) is achieved. Once the voltage is turned off, ultra-fast cooling (>10°C/s) is autonomously achieved through heat dissipation to the surrounding environment, without the need for auxiliary cooling methods. Compared to traditional PCR instruments, where heat is transferred from the heating material to the aluminum substrate, then through a thin layer of air, and finally to the disc-type nucleic acid detection microfluidic chip, this approach offers higher heat transfer efficiency, reduced heat loss, and faster heating and cooling rates. By eliminating the multi-step heat transfer pathways in traditional PCR instruments, the heat transfer efficiency is effectively improved, further enhancing the reaction speed.

The present invention also provides a preferred control method for the aforementioned detection. Specifically, the control method includes controlling the power-on/power-off time (or power supply voltage) of the graphene heating device during the thermal cycling process. This control of the power-on/power-off time (or power supply voltage) is implemented by the control module of the power supply, enabling precise control of heating and cooling under varying environmental conditions (such as temperature and wind speed) for 45 cycles.

As described above, the present invention provides a novel ultra-fast heating and cooling chip based on laser-induced graphene material, which can autonomously achieve ultra-fast temperature changes. The chip is fabricated using laser-induced processing, enabling batch production at low cost. It can improve the temperature control module of current PCR instruments, reduce costs, and enhance the speed of PCR reactions. Furthermore, this ultra-fast heating and cooling chip based on laser-induced graphene material can be effectively applied in the field of point-of-care testing, thereby advancing PCR technology to truly achieve on-site rapid detection.

### Examples

The following examples illustrate the present invention by way of example, but the present invention is not limited thereto. The scope of the present invention is limited only by the claims.

### General Preparation Procedure for Laser-Induced Graphene

In an air atmosphere, a polyimide film substrate with a thickness of 100 µm was scanned at a rate of 100 mm/s using lasers with different fluences: 5.5 J/cm², 5.8 J/cm², 10 J/cm², 15 J/cm², 20 J/cm², 25 J/cm², 30 J/cm², 36 J/cm², 38 J/cm², 40 J/cm², 46 J/cm², 50 J/cm², 60 J/cm², 70 J/cm², and 80 J/cm². The localized heat generated by laser irradiation carbonized the surface of the precursor material, thereby forming graphene.

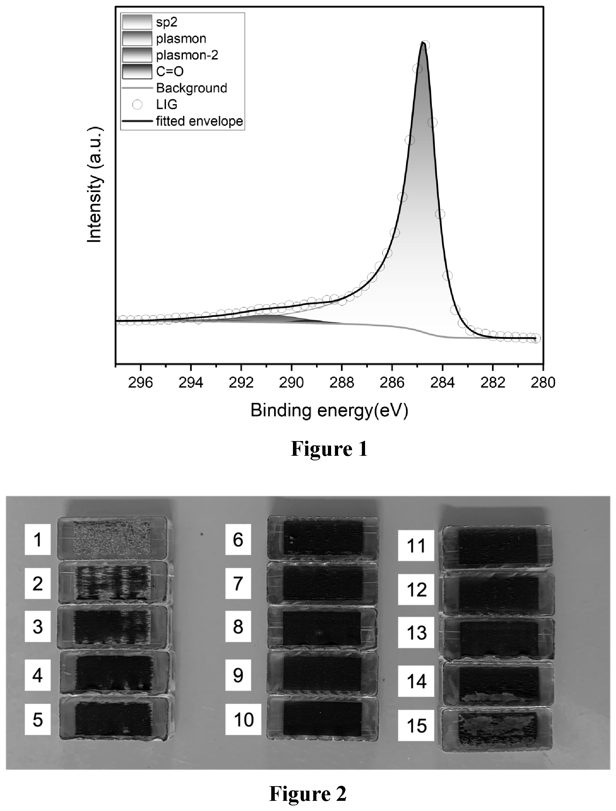

Figure 1 shows the XPS C1s peak fitting of the graphene material prepared by the method of the present invention (with a laser fluence of 36 J/cm²). As can be seen from Figure 1, the resulting material exhibits a significant sp² peak, indicating the formation of a graphene structure.

Figure 2 shows the surface morphology of graphene induced by lasers with fluences of 5.5 J/cm², 5.8 J/cm², 10 J/cm², 15 J/cm², 20 J/cm², 25 J/cm², 30 J/cm², 36 J/cm², 38 J/cm², 40 J/cm², 46 J/cm², 50 J/cm², 60 J/cm², 70 J/cm², and 80 J/cm² (samples 1-15).

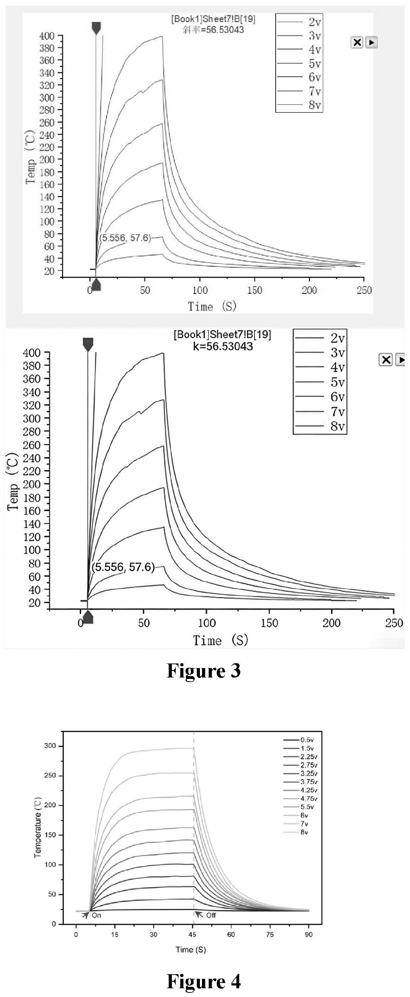

Figure 3 shows the heating curves of the graphene material induced by a laser with a fluence of 40 J/cm² under different voltages, indicating that the maximum surface temperature can reach 398.5°C (all temperature curve values in Figures 3-5 and 7-11 were directly measured on the surface of the heating material or inside the liquid using OMEGA^{™} ultrafine bare thermocouples).

Figure 4 shows the heating curves of the graphene material induced by a laser with a fluence of 36 J/cm² under different voltages, exhibiting the maximum heating rate of 68.5°C/s.

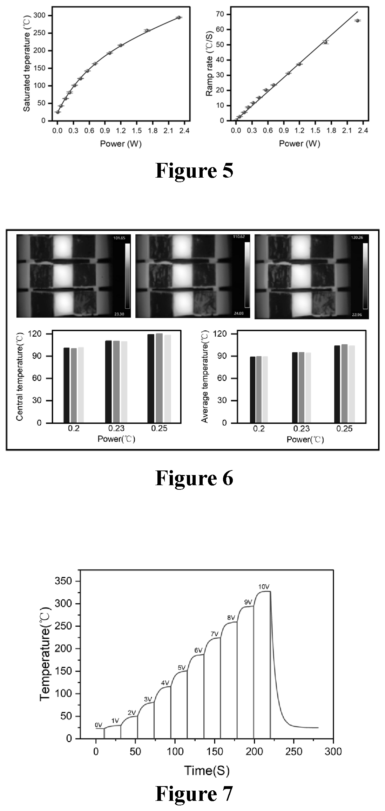

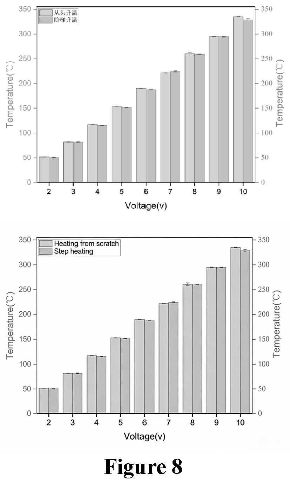

Figure 5 demonstrates a clear functional relationship between the maximum surface temperature and heating rate of laser-induced graphene (with a laser fluence of 36 J/cm²) and the input electrical power. Under the same input power, three independent laser-induced graphene samples (all induced under the same laser fluence of 36 J/cm² and identical conditions) achieved the same surface temperature (Figure 6). This indicates that the temperature of laser-induced graphene can be conveniently and rapidly regulated by adjusting the input electrical energy density. Due to its extremely high electrothermal conversion efficiency, as well as its low specific heat capacity and multilayer porous structure, the rate of converting electrical energy into thermal energy is very fast. This also enables not only convenient but also rapid temperature regulation of laser-induced graphene. Figure 7 illustrates that during continuous heating, with the input voltage increasing in increments of 1 V, the surface temperature of laser-induced graphene (with a laser fluence of 36 J/cm²) exhibits a rapid heating response (the infrared images in Figure 6 and the maximum and average surface temperatures of the heating material were obtained using a FLIR infrared camera). Moreover, under dynamic temperature changes, the real-time surface temperature is nearly identical to the temperature achieved during stepwise heating from the beginning (Figure 8), demonstrating the stable temperature regulation capability of laser-induced graphene through electrical power control.

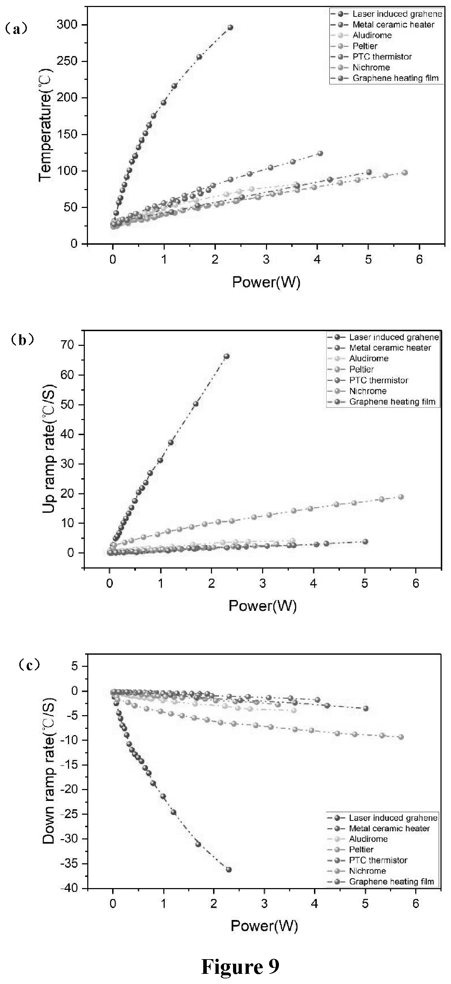

Laser-induced graphene also exhibits rapid cooling capabilities. Due to its low input power and surface structure characterized by a loose and porous morphology (a unique microstructural advantage of laser-induced graphene compared to graphene formed by traditional chemical deposition or surface growth), heat can be quickly dissipated after power is cut off. The maximum natural cooling rate reaches 36°C/s, far exceeding that of traditional electrothermal materials (Figure 9, laser fluence of 36 J/cm²).

During prolonged heating and cooling cycles, laser-induced graphene maintains stable performance. Figure 10 shows the heating and cooling rates and the achievable temperature levels over 100 heating and cooling cycles, remaining stable throughout each cycle (laser fluence of 36 J/cm²).

In summary, leveraging the efficient electrothermal conversion capability of laser-induced graphene, its rapid and precise temperature response under applied voltage, fast temperature change rates, and efficient heat transfer properties, precise regulation of liquid temperature can be achieved, meeting the stringent temperature accuracy requirements of the PCR process. Figure 11 demonstrates that under simple voltage input modulation, the temperature of laser-induced graphene (laser fluence of 36 J/cm²) (red) undergoes regular heating and cooling cycles, enabling the PCR solution temperature (blue) to rapidly alternate between 60°C and 95°C, thereby completing the PCR process.

The above descriptions are merely some implementations of the present application. For those skilled in the art, several modifications and improvements can be made without departing from the creative concept of the present application, all of which fall within the protection scope of the present application.

## Claims

1. A method for preparing laser-induced graphene, comprising: reducing a precursor material layer using laser-induced processing.

2. The method according to claim 1, comprising: providing a carbon-containing precursor material layer, scanning the surface of the carbon-containing precursor material layer with a laser, wherein the localized heat generated by laser irradiation carbonizes the surface of the precursor material, thereby forming graphene;
preferably, the precursor material layer is located on a substrate material layer;
preferably, the precursor material layer and the substrate material layer are the same material integrated as one piece, or are separate different materials;
preferably, the precursor material is selected from polyimide, phenolic resin, polyethyleneimine, lignocellulose, or wood;
preferably, the laser used is selected from a carbon dioxide laser or a femtosecond laser;
preferably, the laser scanning rate is 0.5-500 mm/s, preferably 20-300 mm/s, and more preferably 50-150 mm/s;
preferably, the laser fluence for the laser-induced processing is greater than or equal to 5.5 J/cm², preferably greater than 5.5 J/cm², more preferably 5.8-80 J/cm², even more preferably 5.8-60 J/cm², and most preferably 5.8-50 J/cm²;
preferably, the substrate material is selected from polyimide material, polyimide film, polyimide tape, phenolic resin, polyethyleneimine, lignocellulose, or wood.

3. Laser-induced graphene obtained by the method according to claim 1 or 2.

4. Use of the laser-induced graphene according to claim 3 in PCR detection.

5. A laser-induced graphene PCR detection device, comprising a PCR reaction chamber and a graphene heating device.

6. The laser-induced graphene PCR detection device according to claim 5, wherein the graphene heating device comprises a laser-induced graphene layer and a substrate material layer;
preferably, the laser-induced graphene layer is located on the surface of the substrate material layer and has a thickness of 1-120 µm, preferably 2-100 µm, 3-80 µm, or 5-60 µm.

7. The laser-induced graphene PCR detection device according to claim 5 or 6, wherein the laser-induced graphene layer is prepared by reducing a precursor material layer using laser-induced processing, and the precursor material layer is located on the surface of a substrate material layer, preferably, the laser fluence for the initial formation of laser-induced graphene is 5.5 J/cm².

8. The laser-induced graphene PCR detection device according to any one of claims 5 to 7, wherein the laser used is selected from a carbon dioxide laser or a femtosecond laser, and the substrate material layer is selected from polyimide material, polyimide film, polyimide tape, phenolic resin, polyethyleneimine, lignocellulose, or wood;
preferably, the detection device further comprises a power supply and a power supply control module, wherein the power supply control module controls the power supply to perform power-on/power-off cycling operations on the graphene heating device;
preferably, the graphene heating device is in contact with the PCR reaction chamber, heats the PCR reaction chamber when powered on, and cools the PCR reaction chamber when powered off, thereby achieving heating/cooling cycles of the PCR reaction chamber.

9. A laser-induced graphene PCR detection device, **characterized by** comprising a PCR reaction chamber, a graphene heating device, a power supply, and a power supply control module, wherein the graphene heating device is connected to the power supply, and the power supply is controlled by the power supply control module; the power supply control module controls the power supply to perform power-on/power-off cycling operations on the graphene heating device, and the graphene heating device is in contact with the PCR reaction chamber, heating the PCR reaction chamber when powered on and cooling the PCR reaction chamber when powered off, thereby achieving heating/cooling cycles of the PCR reaction chamber.

10. The laser-induced graphene PCR detection device according to claim 9, **characterized in that** the graphene heating device comprises the laser-induced graphene layer according to claim 4;
preferably, the power supply is a low-voltage DC power supply;
preferably, the power supply includes, but is not limited to, a battery, a rechargeable battery, a mobile power source, a mobile phone, a power supply unit, or a 220V power source;
preferably, the graphene heating device is in the form of a sheet;
preferably, the graphene heating device comprises a laser-induced graphene layer and a substrate material layer, wherein the laser-induced graphene layer is located on the surface of the substrate material layer and has a thickness of 1-120 µm, preferably 2-100 µm, 3-80 µm, or 5-60 µm;
preferably, the PCR detection device is a point-of-care testing device or a high-throughput benchtop detection device;
preferably, the PCR reaction chamber is a disc-type PCR detection chip, and the disc-type PCR detection chip comprises an upper sample introduction layer and a lower detection layer:
preferably, the sample introduction layer is comprised in a top cover layer, and the detection layer is comprised in a liquid storage pool layer.

11. A method for performing PCR detection using the detection device according to claim 9 or 10, **characterized in that** a power supply control module is used to control the power supply to perform power-on/power-off cycling operations on the graphene heating device, thereby subjecting the PCR reaction chamber to 1-120 thermal cycles, preferably 1-80 cycles, more preferably 1-50 cycles, for example, 45 cycles.

12. A PCR detection chip, comprising an upper top cover layer and a lower liquid storage pool layer, wherein the top cover layer is rotatable relative to the liquid storage pool layer; the top cover layer comprises a sample introduction port for adding a detection sample, and an adsorbent material for adsorbing the detection sample is provided below the sample introduction port;
the liquid storage pool layer at least comprises a sample addition pool, an amplification reaction pool, and a product detection pool, wherein during the rotation of the top cover layer, the adsorbent material sequentially enters the sample addition pool, the amplification reaction pool, and the product detection pool, and a test strip introduction hole is provided in the product detection pool.

13. The PCR detection chip according to claim 12, **characterized in that** the top cover layer and the liquid storage pool layer are fastened together to form a sealed space as a whole, and both the top cover layer and the liquid storage pool layer are circular;
preferably, the top cover layer is provided with a rotating handle that can be turned to achieve its rotation;
preferably, the product detection pool is sealed with a sealing material, and the sealing material can melt at a certain temperature, allowing the liquid in the product detection pool to fall; preferably, the sealing material is paraffin, which can melt at 60-90°C;
preferably, a test strip groove for adding a detection test strip is provided below the liquid storage pool layer, and one end of the test strip groove has a through hole serving as an observation window;
preferably, at least one washing pool is further included between the sample addition pool and the amplification reaction pool; preferably, there are two washing pools, including a first washing pool and a second washing pool;
preferably, relative rotation between the top cover layer and the liquid storage pool layer is achieved through a circular slide track;
preferably, the adsorbent material is cellulose filter paper;
preferably, the materials of the top cover layer and the liquid storage pool layer are selected from PMMA, epoxy resin, PVC, or PC;
the concave-convex structures of the slide track and the reaction pools are fabricated using laser etching technology, or the structures are etched on a substrate and then molded with PDMS;
preferably, the first washing pool and the second washing pool are respectively loaded with washing buffer, the amplification reaction pool is loaded with nucleic acid amplification reaction buffer or PCR reaction reagents, and the product detection pool is loaded with running buffer;
preferably, the surface of the top cover layer is marked correspondingly according to the sample addition pool, the first washing pool, the second washing pool, and the amplification reaction pool of the liquid storage pool layer;
preferably, the top cover layer and the liquid storage pool layer are subjected to surface hydrophobic treatment, i.e., the top cover layer and the liquid storage pool layer are treated with trichloro (1H,1H,2H,2H-perfluorooctyl) silane reagent in a vacuum for three hours, allowing the trichloro (1H,1H,2H,2H-perfluorooctyl) silane to form a self-assembled monolayer on the surfaces of the top cover layer and the liquid storage pool layer.

14. A method for nucleic acid detection using the chip according to claim 12 or 13, **characterized by** comprising the following steps:
Step 1: Rotate the sample introduction port of the top cover layer to a position above the sample addition pool of the liquid storage pool layer, use a pipette to inject a certain amount of body fluid onto the surface of the adsorbent material through the sample introduction port, and wait for a certain period of time for the body fluid to dry;
Step 2: Use a finger to turn the rotating handle of the top cover layer, moving the adsorbent material to the first washing pool to wash away impurities on the surface of the adsorbent material, wherein in this step, turning the rotating handle back and forth can accelerate the washing process;
Step 3: Turn the rotating handle of the top cover layer again to move the adsorbent material to the second washing pool for further washing, wherein in this step, turning the rotating handle back and forth can accelerate the washing process;
Step 4: Turn the rotating handle of the top cover layer to move the adsorbent material to the amplification reaction pool; then, turn on the heating device to raise the temperature to a certain level, allowing the reaction buffer in the amplification reaction pool and the nucleic acids on the adsorbent material to undergo an amplification reaction, wherein the amplification reaction includes, but is not limited to, isothermal amplification reactions and PCR reactions;
Step 5: After the amplification reaction is complete, rotate again to move the adsorbent material to the product detection pool, wherein the amplified products on the adsorbent material are absorbed by the detection test strip through the connecting hole; observe the results with the naked eye through the observation window on the test strip groove.

15. A disc-type nucleic acid detection microfluidic chip, **characterized by** comprising an upper top cover layer and a lower liquid storage pool layer, wherein the top cover layer and the liquid storage pool layer are fastened together to form a sealed space as a whole, both the top cover layer and the liquid storage pool layer are circular, and the top cover layer is rotatable relative to the liquid storage pool layer; the top cover layer comprises a sample introduction port for adding a detection sample, and an adsorbent material for adsorbing the detection sample is provided below the sample introduction port; the top cover layer is provided with a rotating handle that can be turned to achieve its rotation;
the liquid storage pool layer at least comprises a sample addition pool, an amplification reaction pool, and a product detection pool, wherein during the rotation of the top cover layer, the adsorbent material sequentially enters the sample addition pool, the amplification reaction pool, and the product detection pool, a test strip introduction hole is provided in the product detection pool and is sealed with a sealing material, which can melt at a certain temperature, allowing the liquid in the product detection pool to fall, wherein
a test strip groove for adding a detection test strip is provided below the liquid storage pool layer, and one end of the test strip groove has a through hole serving as an observation window.

16. The disc-type nucleic acid detection microfluidic chip according to claim 15, **characterized in that**:
at least one washing pool is further included between the sample addition pool and the amplification reaction pool; preferably, there are two washing pools, including a first washing pool and a second washing pool;
preferably, relative rotation between the top cover layer and the liquid storage pool layer is achieved through a circular slide track;
preferably, the sealing material is paraffin, which can melt at 60-90°C;
preferably, the first washing pool and the second washing pool are respectively loaded with washing buffer, the amplification reaction pool is loaded with nucleic acid amplification reaction buffer or PCR reaction reagents, and the product detection pool is loaded with running buffer;
preferably, the surface of the top cover layer is marked correspondingly according to the sample addition pool, the first washing pool, the second washing pool, and the amplification reaction pool of the liquid storage pool layer;
preferably, the top cover layer and the liquid storage pool layer are subjected to surface hydrophobic treatment, i.e., the top cover layer and the liquid storage pool layer are treated with trichloro (1H,1H,2H,2H-perfluorooctyl) silane reagent in a vacuum for three hours, allowing the trichloro (1H,1H,2H,2H-perfluorooctyl) silane to form a self-assembled monolayer on the surfaces of the top cover layer and the liquid storage pool layer.

17. A method for nucleic acid detection using the chip according to claim 15 or 16, **characterized by** comprising the following steps:
Step 1: Rotate the sample introduction port of the top cover layer to a position above the sample addition pool of the liquid storage pool layer, use a pipette to inject a certain amount of body fluid onto the surface of the adsorbent material through the sample introduction port, and wait for a certain period of time for the body fluid to dry;
Step 2: Use a finger to turn the rotating handle of the top cover layer, moving the adsorbent material to the first washing pool to wash away impurities on the surface of the adsorbent material, wherein in this step, turning the rotating handle back and forth can accelerate the washing process;
Step 3: Turn the rotating handle of the top cover layer again to move the adsorbent material to the second washing pool for further washing, wherein in this step, turning the rotating handle back and forth can accelerate the washing process;
Step 4: Turn the rotating handle of the top cover layer to move the adsorbent material to the amplification reaction pool, then, turn on the heating device to raise the temperature to a certain level, allowing the reaction buffer in the amplification reaction pool and the nucleic acids on the adsorbent material to undergo an amplification reaction, wherein the amplification reaction includes, but is not limited to, isothermal amplification reactions and PCR reactions;
Step 5: After the amplification reaction is complete, rotate again to move the adsorbent material to the product detection pool, wherein in the amplified products on the adsorbent material are absorbed by the detection test strip through the connecting hole; observe the results with the naked eye through the observation window on the test strip groove.

18. A detection device comprising the disc-type nucleic acid detection microfluidic chip according to claim 15 or 16, **characterized in that** it comprises a graphene heating device, a power supply, and a power supply control module, wherein the graphene heating device is connected to the power supply, and the power supply is controlled by the power supply control module; the power supply control module controls the power supply to perform power-on/power-off cycling operations on the graphene heating device, and the graphene heating device is in contact with the disc-type nucleic acid detection microfluidic chip, heating the disc-type nucleic acid detection microfluidic chip when powered on and cooling the disc-type nucleic acid detection microfluidic chip when powered off, thereby achieving heating/cooling cycles of the disc-type nucleic acid detection microfluidic chip.

19. The detection device according to claim 18, wherein the graphene heating device comprises the laser-induced graphene according to claim 4.
